# EUROPEAN PATENT SPECIFICATION

(11) **EP 4 153 037 B1**
(45) Date of publication and mention of the grant of the patent: **29.10.2025**
(21) Application number: 21725820.1
(22) Date of filing: 03.05.2021
(51) Int. Cl.: A61B 5/01, A61B 5/145, A61B 5/00

(54) **SYSTEM FOR COMPARING THE PHYSIOLOGICAL PARAMETERS OF A PATIENT**
SYSTEM ZUM VERGLEICHEN DER PHYSIOLOGISCHEN PARAMETER EINES PATIENTEN
SYSTÈME DE COMPARAISON DES PARAMÈTRES PHYSIOLOGIQUES D'UN PATIENT

(30) Priority: 21.05.2020 IT 202000011881
(43) Date of publication of application: 29.03.2023
(73) Proprietor: Skin Plastic Lab S.r.l., 73024 Maglie (LE) (IT)
(72) Inventor: PERTICAROLI, Stefano, 73024 Maglie (LE) (IT); PAPA, Giovanni, 73024 MAGLIE (LE) (IT)
(74) Representative: Fiammenghi, Eva
(86) International application number: PCT/IB2021/053668
(87) International publication number: WO 2021/234484

(56) References cited:
- WO-A1-2019/048624
- US-A1- 2006 047 218
- US-A1- 2013 261 494

## Description

### Field of the art

The present invention operates in the field of electronic devices for aiding the diagnostic, medical and more generally health field.

In particular the system of the present invention is adapted to be used for monitoring the vitality of the skin layers, and in a particularly convenient embodiment, in post-operation phase for verifying that a wound is correctly healing.

### Prior art

The temperature of the skin, intended as member constituted by different tissues and sprayed by blood vessels, is a fundamental indicator in the determination of the vitality of the skin itself.

There is a correlation, defined and proven by numerous scientific articles, between the temperature variations of the skin and pathologies which affect the skin and also the deep circulation in some cases, such as those relative to the limbs. For example, temperature gradients in skin areas are observed in patients with vascular disorders which have an irregular blood flow in the affected zones. The current systems existing for thermographic imaging, capable of detecting temperature gradients and/or of monitoring blood flow variations of tissues and/or organs, are based on infrared thermography or on the perfusion of tracers.

In the case of infrared thermography, the method for transmitting heat between the source, represented by a tissue and/or organ volume, is detected by means of irradiation. This type of transmission and detection of the temperature is intrinsically non-selective since it is capable of determining the temperature of a body not in contact with the sensor, limiting the discrimination capacity at the average temperature of a volume with accuracy levels at most on the order of tenths of centigrade degrees.

In the case of the perfusion of intravenous tracers for the determination of the blood flow variations, indocyanine green or fluorescein are considered. This type of transmission and detection of the information relative to the blood flows is based on radiations in the optical or near-optical spectrum (ultraviolet), by means of auxiliary machinery, such as microscopes or microchambers sensitive to specific wavelengths. The latter two methods, even if they have greater accuracies, are invasive treatments for the patient since the tracers must be perfused and do not give information directly correlated to the temperature. Finally, they do not allow the monitoring of the magnitudes of interest in real time and for a prolonged time period.

In the same field of application as the present patent application, several patents are already present, such as for example the patent US2001/206655 which describes a bandage that incorporates a matrix of temperature sensors fixable to the body of a patient. The bandage can contain electronic processing components and a transmitter. Another patent WO2009/144615 claims a device for the electrical treatment of a wound which comprises: a flexible substrate, provided with a matrix of electrodes, adapted to be attached to the skin wound; means for determining the presence of the wound and its perimeter; means for controlling a liquid load between the matrix and the wound and means for applying an electrical voltage between the wound and the surrounding skin. Other known documents claim the use of temperature sensors for sanitary and diagnostic purposes. In particular the patent US2018/183794 describes a method for determining the emergence of an ulcer on a foot of a patient starting from the detections carried out by a plurality of temperature sensors situated on a device configured for receiving at least a foot.

Indeed, patent US2016/183794 describes a temperature detector bandage which includes: a sealed and flexible battery comprising a printed electrochemical cell, a flexible circuit which includes a microprocessor, a temperature sensor, a wireless transmitter and an antenna for transmitting the detected data to a remote device.

US2006/047218 describes a monitoring system wearable by a user. At least a pair of sensors (temperature, impedance) is provided in association with a support member in the form of a bandage, drain or other structure. Monitoring facilities may have a stand-alone utility or be linked to a processor or data logger to enable various functions.

US2013/261494 relates to a method for monitoring at least one foot of a patient and provides an open platform to receive at least one foot. The open platform has at least one temperature sensor for generating a plurality of temperature data values after receiving the foot.

WO2019/048624 discloses methods of inspection and fabrication of flexible printed circuit boards and flexible sensor sheets, and apparatuses such as wound dressing components using the same.

The technical problem raised by the Applicant regards the fact that the detection of the temperature data only in the vicinity of the wound could be altered in the case of an increase or a decrease of the temperature throughout the entire body of the patient. In addition, the limitation of the indications of correct or poor healing to only the temperature data seems to provide an incomplete and easily erroneous diagnosis.

From the study of the prior art, neither documents nor devices have been found which are capable of executing analyses of all the important physiological parameters for ascertaining the correct healing of a wound and the correct blood circulation, both in proximity to a wound and in portions of healthy skin. Nor have there been mentions of devices that can compare the physiological parameters detected in the portion to be diagnosed with the general parameters of the patient, detected from body portions that are far from or extraneous to the wound or to the presumed circulatory problem.

Object of the present invention is, therefore, that of filling this gap of the prior art, by proposing a new and innovative system for comparing the physiological parameters of a patient which overcomes the critical factors encountered and which allows an accurate diagnosis of the state of healing of a wound and of possible circulatory problems.

### Description of the invention

According to the present invention, a system is described for comparing the physiological parameters of a patient which resolves the abovementioned problems.

Such system advantageously consists of a pair of detection devices, a main one and at least a secondary, which are adapted to detect the same physiological parameters in two distinct portions of the body of a patient.

In more detail, the primary detection device is adapted to be positioned at a skin portion, healthy or damaged, regarding which it is desired to monitor the perfusion state of a wound or regarding which it is desired to verify the presumed presence of circulatory disturbances.

The detected parameters will be at least those of skin temperature and, in some still more advantageous embodiments, also the pH and the skin oxygenation will be detected through dedicated sensors.

In order to avoid considering possible irregular parameters, which however are not only localized in the area to be diagnosed, but rather extended over the entire body, the present invention advantageously comprises at least a second detection device to be positioned in a healthy portion of the body of the patient, possibly far from that to be diagnosed.

Said primary detection device, entering into more detail, is constituted by a first printed circuit and by a second printed circuit arranged on a flexible support, adapted to be placed at the first skin portion to be diagnosed, healthy or damaged, of a patient, being adapted to any curve of the body. The first printed circuit is that which comprises at least a matrix of temperature sensors and possibly also pH and skin oxygenation sensors. The detected data is sent to at least a microcontroller integrated in said second printed circuit.

The secondary detection device or the secondary detection devices are configured to be equivalent to the primary one.

The microcontrollers integrated in all the detection devices send the data to a display device, wired or wireless, provided with at least a screen adapted to allow the display of the data detected by said primary detection device and by said at least a secondary detection device.

The primary detection device can be advantageously enlarged, i.e. the area over which the physiological parameters are detected can be extended.

By means of a connection cable arranged on each side of the perimeter of said primary detection device, an additional detection portion can be engaged with a corresponding connection cable; each additional detection portion can be aggregated at a side of the perimeter of said primary detection device. Each additional detection portion advantageously comprises a printed circuit with a matrix of sensors adapted to detect the same physiological parameters detected by the matrix of sensors comprised in the first printed circuit of the primary detection device. The printed circuit of each additional portion is advantageously configured for being connected to the printed circuit of the primary detection device by means of said connection cable, sending the detected data to the microcontroller and then to the display device.

Also the secondary detection device can be provided with the same cables of connection with the corresponding additional detection portions.

The advantages of the present invention are particularly important for post-operation use, for monitoring the healing of wounds. In addition, the detection of the aforesaid physiological parameters can be equally useful and advantageous for the diagnosis of disturbances of the venous and arterial circulation, which alter the skin temperature.

Possible further medical applications and uses of the present industrial invention patent application regard numerous medical science fields including the following, which are indicated as non-limiting examples:
- plastic surgery, for carrying out the evaluation of the vitality of the skin flap in substance loss reconstruction;
- traumatology, for carrying out the correct evaluation of the laceration-contusion wounds;
- burns, for obtaining an improved evaluation of the deep burns based on the skin vitality;
- diabetology, in the precise and circumscribed evaluation of the vital tissue, before amputation of the diabetic foot;
- in surgery, in the evaluation of the vascularization at the margins of the suture, both pre- and post-operation.

The advantages offered by the present invention are evident in light of the description set forth up to now and will be even clearer due to the enclosed figures and to the relative detailed description.

### Description of the figures

The invention will be described hereinbelow in at least a preferred embodiment by way of a non-limiting example with the aid of the enclosed figures, in which:
- FIGURE 1 schematically shows all the components of the system, object of the present invention, in the embodiment in which the communication of the data to the display device 50 occurs in wireless form.

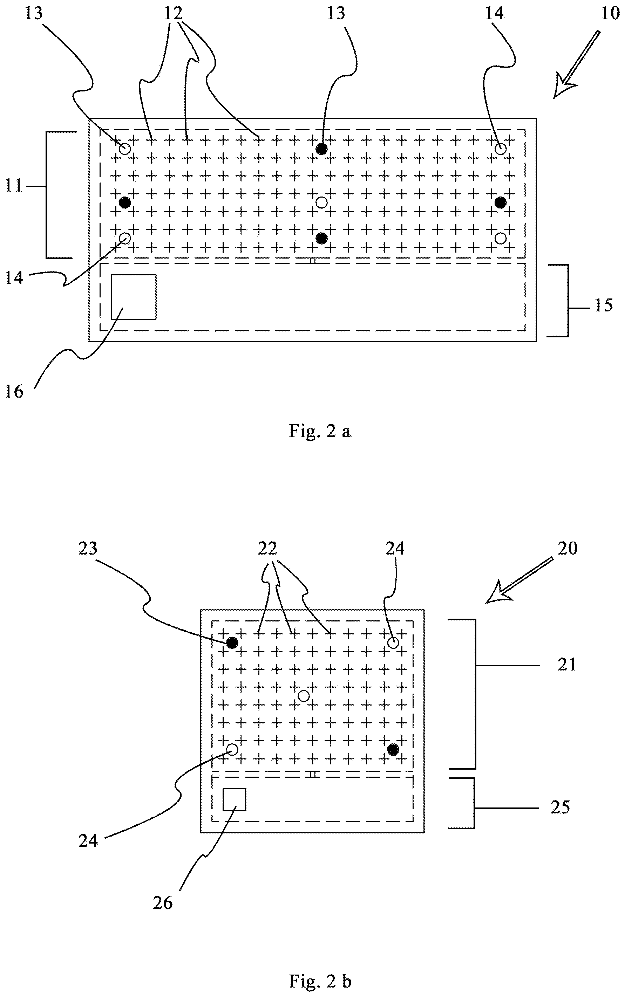

- FIGURE 2 illustrates in more detail the various electronic and sensor components of the primary detection device 10 (Fig. 2a) and of the secondary detection devices 20-20' (Fig. 2b).

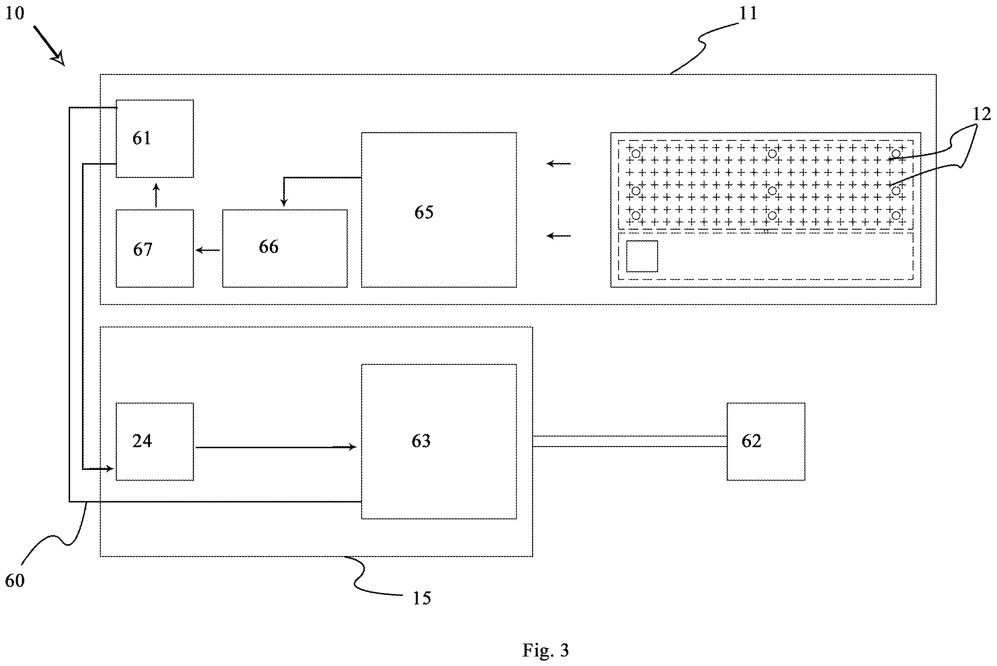

- FIGURE 3 shows in more detail one of the possible embodiments of the printed circuits 11-15 of the detection devices 10-20-20'.

### Detailed description of the invention

The present invention will now be illustrated as a merely exemplifying but non-limiting or non-constraining embodiment, with reference to the figures which illustrate several embodiments relative to the present inventive concept.

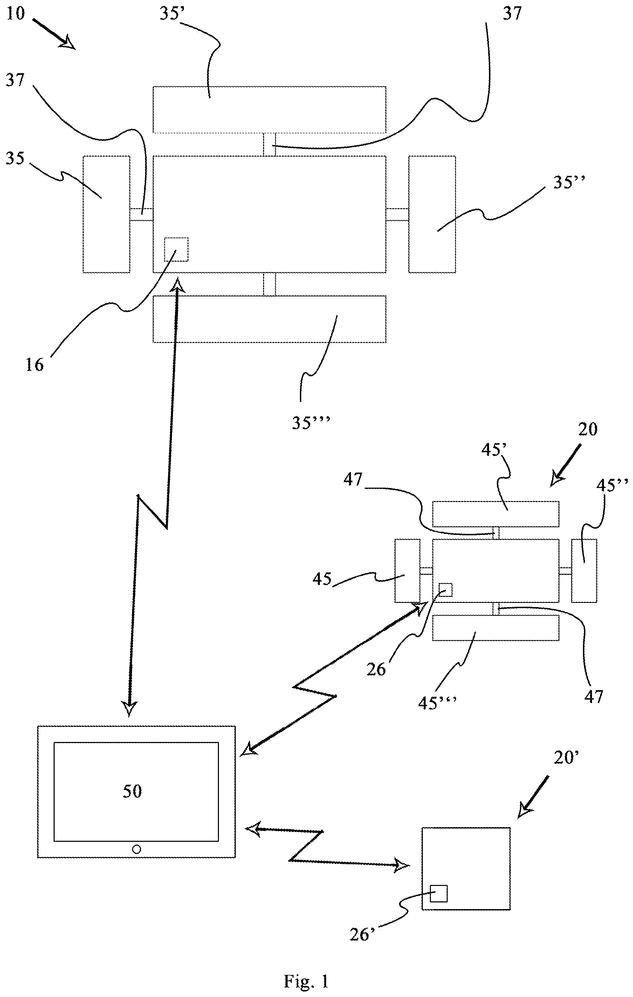

With reference to FIG. 1, the essential components of the present invention are shown, i.e.:
- a primary detection device 10 constituted by a first printed circuit 11 and a second printed circuit 15 arranged on a flexible support made of polyimide so as to be adapted to any curve of the patient's body. Such primary detection device 10 is adapted to be placed at a first skin portion to be diagnosed, healthy or damaged, of a patient.
- a first secondary detection device 20 constituted by a first printed circuit 21 and a second printed circuit 25 arranged on a flexible support made of polyimide so as to be adapted to any curve of the body of the patient. Such secondary detection device 20 is adapted to be placed at a second skin portion to be compared, said second portion being healthy;
- a display device 50 connected in a wireless manner to the microcontrollers 16-26 26' integrated in the detection devices 10-20-20'. The display device 50 is provided with at least a screen in order to allow the display of the data detected by the connected detection devices 10-20-20', executing a comparison of the data detected on a healthy portion and that detected on the damaged portion or in any case on the portion to be diagnosed.

The present system can also comprise at least a second secondary detection device 20' configured like the first secondary detection device 20 described above.

From Fig. 1, one also infers the possibility to extend the area detected by each detection device 10-20-20' by means of additional portions 35-35'-35"-35‴; 45-45'-45"-45‴ connected to the respective central portion by means of a connection cable 37-47 for each side of the perimeter of said detection devices 10-20-20'. Each additional detection portion 35-35'-35"-35‴; 45-45'-45"-45‴ comprises a printed circuit with a matrix of sensors adapted to detect the same physiological parameters detected by the matrix of sensors comprised in the first printed circuit 11-21 of the central portion of said detection device 10-20-20'. The printed circuit of each additional portion 35-35'-35"-35‴; 45-45'-45"-45‴ is configured for being connected to the printed circuit of the central portion of the detection device 10-20-20'-... by means of said connection cable 37-47, sending the detected data to the respective microcontroller 16-26 and, finally, to the display device 50.

Entering into more detail in the technical operation of the printed circuits 11-15-21-25 of the detection devices 10-20-20', these are preferably configured as follows.

A first printed circuit 11-21 comprises at least a matrix of temperature sensors 12-22, of acidity sensors (pH) 13-23 and of skin oxygenation sensors 14-24, as these are the physiological parameters which indicate the vitality of the skin. Said matrix comprising, preferably, 16 lines by 32 columns.

A connector 61, in which a flexible connection cable 60 is inserted, connects the first printed circuit 11-21 to the second printed circuit 15-25.

The second printed circuit 15-25 comprises:
- a MCU 63 adapted to select said sensors 12-22-13-23-14-24 one at a time, sending on a bus the number of the line and of the column to be selected and adapted to receive the measurement of the sensor by reading the output of a digital analog converter 64;
- selection devices 65 adapted to activate the line and the corresponding column;
- a transistor in series with each sensor 12-22-13-23-14-24 adapted to select the corresponding sensor 12-22-13-23-14-24;
- a NTC resistor in order to connect the power supply to a resistive divider 66;
- said resistive divider 66 adapted to acquire the current from said NTC resistor and generate a voltage;
- a LNA 67 adapted to scale and translate the possible output voltages from said resistive divider 66 in order to fully exploit the input dynamics of said digital analog converter 64.
- USB interface means 62 adapted to transmit the digitized information to other peripheral devices.

## Claims

1. System for comparing the physiological parameters of a patient, adapted to acquire, process and compare detected data relative to the parameters of skin vitality in at least a pair of distinct portions of the body of a patient; said system being **characterized in that** it comprises at least:
- one primary detection device (10) constituted by a first printed circuit (11) and a second printed circuit (15) arranged on a flexible support, adapted to be placed at a first skin portion to be diagnosed, healthy or damaged, of a patient; said first printed circuit (11) comprising at least a matrix of temperature sensors (12) adapted to send the detected temperature data to at least a microcontroller (16) integrated in said second printed circuit (15);
- at least a secondary detection device (20) constituted by a first printed circuit (21) and a second printed circuit (25) arranged on a flexible support, adapted to be placed at a second skin portion, healthy, of a patient in order to detect the same data as said first skin portion; said first printed circuit (21) comprising at least a matrix of temperature sensors (22) adapted to send the detected temperature data to at least a microcontroller (26) integrated in said second printed circuit (25);
- a display device (50) connected to said microcontrollers (16-26), wired or wireless, provided with at least a screen adapted to allow the display of the detected data by said primary detection device (10) and by said at least a secondary detection device (20);
at least said primary detection device (10) comprises at least connection cable (37) for each side, adapted to be reversibly engaged with a corresponding cable of an additional detection portion (35-35'-35"-35‴), each of which aggregable at one side of the perimeter of said primary detection device (10); each additional detection portion (35-35'-35"-35‴) comprising a printed circuit with a matrix of sensors adapted to detect the same physiological parameters detected by the matrix of sensors comprised in the first printed circuit (11) of the central portion of said primary detection device (10); said printed circuit of each additional portion (35-35'-35"-35‴) being configured for being connected to the printed circuit of the central portion of the primary detection device (10) by means of said connection cable (37), sending the detected data to the microcontroller (16) of the aforesaid central portion of the primary detection device (10).

2. System for comparing the physiological parameters of a patient, according to the preceding claim 1, **characterized in that** it comprises a plurality of secondary detection devices (20-20'-...) adapted to detect the physiological parameters in a corresponding plurality of skin portions, sending the detected data to said display device (50).

3. System for comparing the physiological parameters of a patient, according to any one of the preceding claims 1 or 2, **characterized in that** each detection device (10-20-20'-...) comprises at least an acidity sensor or matrix of acidity sensors (13-23) adapted to detect the corresponding data regarding the pH of the skin portion on which said detection devices (10-20-20'-...) are positioned, sending the corresponding data to said display device (50).

4. System for comparing the physiological parameters of a patient, according to any one of the preceding claims, **characterized in that** each detection device (10-20-20'-...) comprises at least an oxygenation sensor or matrix of oxygenation sensors (14-24) adapted to detect the corresponding data regarding the skin oxygenation of the skin portion on which said detection devices (10-20-20'-...) are positioned, sending the corresponding data to said display device (50).

5. System for comparing the physiological parameters of a patient, according to any one of the preceding claims, **characterized in that** at least a of said secondary detection devices (20-20'-...) comprises at least connection cable (47) for each side, adapted to be reversibly engaged with a corresponding cable of an additional detection portion (45-45'-45"-45‴), each of which aggregable at one side of the perimeter of said secondary detection device (20-20'-...); each additional detection portion (45-45'-45"-45‴) comprising a printed circuit with a matrix of sensors adapted to detect the same physiological parameters detected by the matrix of sensors comprised in the first printed circuit (21) of the central portion of said secondary detection device (20-20'-...); said printed circuit of each additional portion (45-45'-45"-45‴) being configured for being connected to the printed circuit of the central portion of the secondary detection device (20-20'-...) by means of said connection cable (47), sending the detected data to the microcontroller (26) of the aforesaid central portion of the secondary detection device (20-20'-...).

6. System for comparing the physiological parameters of a patient, according to any one of the preceding claims, **characterized in that** said first printed circuit (11-21) and said second printed circuit (15-25) of each detection device (10-20-20'-...) comprise:
- a connector (61) in which a flexible connection cable (60) is inserted;
- a plurality of sensors (12-22-13-23-14-24) arranged in a matrix of at least 16 lines by 32 columns;
- an MCU (63) adapted to select said sensors (12-22-13-23-14-24) one at a time, sending on a bus the number of the line and of the column to be selected and adapted to receive the measurement of the sensor by reading the output of a digital analog converter (64);
- selection devices (65) adapted to activate the line and the corresponding column;
- a transistor in series with each sensor (12-22-13-23-14-24) adapted to select the corresponding sensor (12-22-13-23-14-24);
- an NTC resistor adapted to connect the power supply to a resistive divider (66);
- said resistive divider (66) adapted to acquire the current from said NTC resistor and generate a voltage;
- an LNA (67) adapted to scale and translate the possible output voltages from said resistive divider (66) in order to fully exploit the input dynamics of said digital analog converter (64).

7. System for comparing the physiological parameters of a patient, according to any one of the preceding claims, **characterized in that** at least said primary detection device (10) comprises USB interface means (62) adapted to transmit the digitized data to other periphery devices.

8. System for comparing the physiological parameters of a patient, according to any one of the preceding claims, **characterized in that** said flexible supports of said detection devices (10-20-20'-...) are made of polyimide so as to be adapted to any curve of the patient's body.

## Patentansprüche

1. System zum Vergleichen der physiologischen Parameter eines Patienten, das dazu ausgelegt ist, detektierte Daten in Bezug auf die Parameter der Hautvitalität in zumindest zwei unterschiedlichen Abschnitten des Körpers eines Patienten zu erfassen, zu verarbeiten und zu vergleichen, wobei das System **dadurch gekennzeichnet ist, dass** es zumindest aufweist:
- eine primäre Detektionseinrichtung (10), die durch eine erste gedruckte Schaltung (11) und eine zweite gedruckte Schaltung (15), die auf einem flexiblen Träger angeordnet sind, gebildet und dazu ausgelegt ist, auf einem ersten zu diagnostizierenden, gesunden oder geschädigten Hautabschnitt eines Patienten platziert zu werden; wobei die erste gedruckte Schaltung (11) zumindest eine Matrix von Temperatursensoren (12), die dazu ausgelegt sind, die detektierten Temperaturdaten an zumindest einen in die zweite gedruckte Schaltung (15) integrierten Mikrocontroller (16) zu senden, aufweist;
- zumindest eine sekundäre Detektionseinrichtung (20), die durch eine erste gedruckte Schaltung (21) und eine zweite gedruckte Schaltung (25), die auf einem flexiblen Träger angeordnet sind, gebildet und dazu ausgelegt ist, auf einem zweiten, gesunden Hautabschnitt eines Patienten platziert zu werden, der gesund ist, um die gleichen Daten wie der erste Hautabschnitt zu detektieren; wobei die erste gedruckte Schaltung (21) zumindest eine Matrix von Temperatursensoren (22), die dazu ausgelegt sind, die detektierten Temperaturdaten an zumindest einen in die zweite gedruckte Schaltung (25) integrierte Mikrocontroller (26) zu senden, aufweist;
- eine drahtgebunden oder drahtlos mit den Mikrocontrollern (16-26) verbundene Anzeigeeinrichtung (50), die mit zumindest einem Bildschirm versehen ist, der dazu ausgelegt ist, die Anzeige der durch die primäre Detektionseinrichtung (10) und durch die zumindest eine sekundäre Detektionseinrichtung (20) detektierten Daten zu ermöglichen;
wobei zumindest die primäre Detektionseinrichtung (10) für jede Seite zumindest ein Verbindungskabel (37) aufweist, das dazu ausgelegt ist, reversibel mit einem entsprechenden Kabel eines zusätzlichen Detektionsabschnitts (35-35'-35"-35‴), von denen jeder an einer Seite des Umfangs der primären Detektionseinrichtung (10) aggregierbar ist, gekuppelt zu werden;
wobei jeder zusätzliche Detektionsabschnitts (35-35'-35"-35"') eine gedruckte Schaltung mit einer Matrix von Sensoren aufweist, die dazu ausgelegt sind, dieselben physiologischen Parameter, die durch die in der ersten gedruckten Schaltung (11) des zentralen Abschnitts der primären Detektionseinrichtung (10) enthaltenen Matrix von Sensoren, detektiert werden, zu detektieren; wobei die gedruckte Schaltung eines jeden zusätzlichen Abschnitts (35-35'-35"-35‴) dazu ausgebildet ist, mittels des Verbindungskabels (37) mit der gedruckten Schaltung des zentralen Abschnitts der primären Detektionseinrichtung (10) verbunden zu werden und die detektierten Daten an den Mikrocontroller (16) des zentralen Abschnitts der primären Detektionseinrichtung (10) zu senden.

2. System zum Vergleichen der physiologischen Parameter eines Patienten gemäß dem vorstehenden Anspruch 1, **dadurch gekennzeichnet, dass** es mehrere sekundäre Detektionseinrichtungen (20-20'-...) aufweist, die dazu ausgelegt sind, die physiologischen Parameter in einer entsprechenden Vielzahl von Hautabschnitten zu detektieren und die detektierten Daten an die Anzeigeeinrichtung (50) zu senden.

3. System zum Vergleichen der physiologischen Parameter eines Patienten gemäß einem der vorstehenden Ansprüche 1 oder 2, **dadurch gekennzeichnet, dass** jede Detektionseinrichtung (10-20-20'-...) zumindest einen Säuresensor oder eine Matrix von Säuresensoren (13-23), die dazu ausgelegt sind, die entsprechenden Daten bezüglich des pH-Werts des Hautabschnitts, auf dem die Detektionseinrichtungen (10-20-20'-...) positioniert sind, zu erfassen und die entsprechenden Daten an die Anzeigeeinrichtung (50) zu senden, aufweist.

4. System zum Vergleichen der physiologischen Parameter eines Patienten gemäß einem der vorstehenden Ansprüche, **dadurch gekennzeichnet, dass** jede Detektionseinrichtung (10-20-20'-...) zumindest einen Sauerstoffsensor oder eine Matrix von Sauerstoffsensoren (14-24), die dazu ausgelegt sind, die entsprechenden Daten bezüglich der Hautoxygenierung des Hautabschnitts, auf dem die Detektionseinrichtungen (10-20-20'-...) positioniert sind, zu erfassen und die entsprechenden Daten an die Anzeigeeinrichtung (50) zu senden, aufweist.

5. System zum Vergleichen der physiologischen Parameter eines Patienten gemäß einem der vorstehenden Ansprüche, **dadurch gekennzeichnet, dass** zumindest eine der sekundären Detektionseinrichtungen (20-20' -...) für jede Seite zumindest ein Verbindungskabel (47) aufweist, das dazu ausgelegt ist, reversibel mit einem entsprechenden Kabel eines zusätzlichen Detektionsabschnitts (45-45'-45"-45‴), von denen jeder an einer Seite des Umfangs der sekundären Detektionseinrichtung (20-20'-...) aggregierbar ist, gekuppelt zu werden; wobei jeder zusätzlicher Detektionsabschnitt (45-45'-45"-45‴) eine gedruckte Schaltung mit einer Matrix von Sensoren aufweist, die dazu ausgelegt sind, dieselben physiologischen Parameter, die durch die in der ersten gedruckten Schaltung (21) des zentralen Abschnitts der sekundären Detektionseinrichtung (20-20'- ...) enthaltenen Matrix von Sensoren detektiert werden, zu detektieren; wobei die gedruckte Schaltung jedes zusätzlichen Abschnitts (45-45'-45"-45‴) dazu ausgebildet ist, mittels des Verbindungskabels (47) mit der gedruckten Schaltung des zentralen Abschnitts der sekundären Detektionseinrichtung (20-20'-...) verbunden zu werden und die detektierten Daten an den Mikrocontroller (26) des vorgenannten zentralen Abschnitts der sekundären Detektionseinrichtung (20-20'- ...) zu senden.

6. System zum Vergleichen der physiologischen Parameter eines Patienten gemäß einem der vorstehenden Ansprüche, **dadurch gekennzeichnet, dass** die erste gedruckte Schaltung (11-21) und die zweite gedruckte Schaltung (15-25) einer jeden Detektionseinrichtung (10-20-20'- ...) aufweisen:
- einen Verbinder (61), in den ein flexibles Verbindungskabel (60) eingeführt ist;
- mehrere Sensoren (12-22-13-23-14-24), die in einer Matrix von zumindest 16 Zeilen mal 32 Spalten angeordnet sind;
- eine MCU (63), die dazu ausgelegt ist, die Sensoren (12-22-13-23-14-24) einzeln auszuwählen, die Nummer der auszuwählenden Zeile und Spalte auf einem Bus zu senden, und dazu ausgelegt ist, die Messung des Sensors durch Lesen des Ausgangs eines Digital-Analog-Wandlers (64) zu empfangen;
- Auswahleinrichtungen (65), die dazu ausgelegt sind, die Zeile und die entsprechende Spalte zu aktivieren;
- einen Transistor in Reihe mit jedem Sensor (12-22-13-23-14-24), der dazu ausgelegt ist, den entsprechenden Sensor (12-22-13-23-14-24) auszuwählen;
- einen NTC-Widerstand, der dazu ausgelegt ist, die Leistungsversorgung mit einem resistiven Teiler (66) zu verbinden;
- wobei der resistive Teiler (66) dazu ausgelegt ist, den Strom von dem NTC-Widerstand zu erfassen und eine Spannung zu erzeugen;
- einen LNA (67), der dazu ausgelegt ist, die möglichen Ausgangsspannungen von dem resistiven Teiler (66) zu skalieren und umzuwandeln, um die Eingangsdynamik des Digital-Analog-Wandlers (64) vollständig auszunutzen.

7. System zum Vergleichen der physiologischen Parameter eines Patienten gemäß einem der vorstehenden Ansprüche, **dadurch gekennzeichnet, dass** die primäre Detektionseinrichtung (10) ein USB-Schnittstellenmittel (62), das dazu ausgelegt ist, die digitalisierten Daten an andere Peripheriegeräte zu senden, aufweist.

8. System zum Vergleichen der physiologischen Parameter eines Patienten gemäß einem der vorstehenden Ansprüche, **dadurch gekennzeichnet, dass** die flexiblen Träger der Detektionseinrichtungen (10-20-20'- ...) aus Polyimid bestehen, um sich an jede Krümmung des Körpers des Patienten anzupassen.

## Revendications

1. Système pour comparer les paramètres physiologiques d'un patient, adapté pour acquérir, traiter et comparer des données détectées relatives aux paramètres de vitalité cutanée dans au moins une paire de portions distinctes du corps d'un patient ; ledit système étant **caractérisé en ce qu'**il comprend au moins :
- un dispositif de détection primaire (10) constitué par un premier circuit imprimé (11) et un deuxième circuit imprimé (15) agencé sur un support flexible, adapté pour être placé au niveau d'une première portion de peau à diagnostiquer, saine ou lésée, d'un patient ; ledit premier circuit imprimé (11) comprenant au moins une matrice de capteurs de température (12) adaptés pour envoyer les données de température détectées à au moins un microcontrôleur (16) intégré dans ledit deuxième circuit imprimé (15) ;
- au moins un dispositif de détection secondaire (20) constitué par un premier circuit imprimé (21) et un deuxième circuit imprimé (25) agencé sur un support flexible, adapté pour être placé au niveau d'une deuxième portion de peau, saine, d'un patient afin de détecter les mêmes données que ladite première portion de peau ; ledit premier circuit imprimé (21) comprenant au moins une matrice de capteurs de température (22) adaptés pour envoyer les données de température détectées à au moins un microcontrôleur (26) intégré dans ledit deuxième circuit imprimé (25) ;
- un dispositif d'affichage (50) connecté auxdits microcontrôleurs (16-26), câblé ou sans fil, pourvu d'au moins un écran adapté pour permettre l'affichage des données détectées par ledit dispositif de détection primaire (10) et par ledit au moins un dispositif de détection secondaire (20) ;
au moins ledit dispositif de détection primaire (10) comprend au moins un câble de connexion (37) pour chaque côté, adapté pour être engagé de manière réversible avec un câble correspondant d'une portion de détection supplémentaire (35-35'-35"-35‴), chacun agrégable au niveau d'un côté du périmètre dudit dispositif de détection primaire (10) ; chaque portion de détection supplémentaire (35-35'-35"-35‴) comprenant un circuit imprimé avec une matrice de capteurs adaptée pour détecter les mêmes paramètres physiologiques détectés par la matrice de capteurs comprise dans le premier circuit imprimé (11) de la portion centrale dudit dispositif de détection primaire (10) ; ledit circuit imprimé de chaque portion supplémentaire (35-35'-35''-35‴) étant configuré pour être connecté au circuit imprimé de la portion centrale du dispositif de détection primaire (10) au moyen dudit câble de connexion (37), envoyant les données détectées au microcontrôleur (16) de ladite portion centrale du dispositif de détection primaire (10).

2. Système pour comparer les paramètres physiologiques d'un patient, selon la revendication 1 précédente, **caractérisé en ce qu'**il comprend une pluralité de dispositifs de détection secondaires (20-20'-...) adaptés pour détecter les paramètres physiologiques dans une pluralité correspondante de portions de peau, envoyant les données détectées audit dispositif d'affichage (50).

3. Système pour comparer les paramètres physiologiques d'un patient, selon l'une des revendications 1 ou 2 précédentes, **caractérisé en ce que** chaque dispositif de détection (10-20-20'-...) comprend au moins un capteur d'acidité ou une matrice de capteurs d'acidité (13-23) adaptés pour détecter les données correspondantes concernant le pH de la portion de peau sur laquelle lesdits dispositifs de détection (10-20-20'-...) sont positionnés, envoyant les données correspondantes audit dispositif d'affichage (50).

4. Système pour comparer les paramètres physiologiques d'un patient, selon l'une des revendications précédentes, **caractérisé en ce que** chaque dispositif de détection (10-20-20'-...) comprend au moins un capteur d'oxygénation ou une matrice de capteurs d'oxygénation (14-24) adaptés pour détecter les données correspondantes concernant l'oxygénation cutanée de la portion de peau sur laquelle lesdits dispositifs de détection (10-20-20'-...) sont positionnés, envoyant les données correspondantes audit dispositif d'affichage (50).

5. Système pour comparer les paramètres physiologiques d'un patient, selon l'une des revendications précédentes, **caractérisé en ce qu'**au moins un desdits dispositifs de détection secondaires (20-20'-...) comprend au moins un câble de connexion (47) pour chaque côté, adapté pour être engagé de manière réversible avec un câble correspondant d'une portion de détection supplémentaire (45-45'-45''-45‴), chacun agrégable au niveau d'un côté du périmètre dudit dispositif de détection secondaire (20-20'-...) ; chaque portion de détection supplémentaire (45-45'-45''-45‴) comprenant un circuit imprimé avec une matrice de capteurs adaptée pour détecter les mêmes paramètres physiologiques détectés par la matrice de capteurs comprise dans le premier circuit imprimé (21) de la portion centrale dudit dispositif de détection secondaire (20-20'-...) ; ledit circuit imprimé de chaque portion supplémentaire (45-45'-45"-45‴) étant configuré pour être connecté au circuit imprimé de la portion centrale du dispositif de détection secondaire (20-20'-...) au moyen dudit câble de connexion (47), envoyant les données détectées au microcontrôleur (26) de ladite portion centrale du dispositif de détection secondaire (20-20'-...).

6. Système pour comparer les paramètres physiologiques d'un patient, selon l'une des revendications précédentes, **caractérisé en ce que** ledit premier circuit imprimé (11-21) et ledit deuxième circuit imprimé (15-25) de chaque dispositif de détection (10-20-20'-...) comprennent :
- un connecteur (61) dans lequel est inséré un câble de connexion flexible (60) ;
- une pluralité de capteurs (12-22-13-23-14-24) agencés dans une matrice d'au moins 16 lignes par 32 colonnes ;
- une MCU (63) adaptée pour sélectionner lesdits capteurs (12-22-13-23-14-24) un à la fois, envoyant sur un bus le numéro de la ligne et de la colonne à sélectionner et adaptée pour recevoir la mesure du capteur en lisant la sortie d'un convertisseur analogique numérique (64) ;
- des dispositifs de sélection (65) adaptés pour activer la ligne et la colonne correspondante ;
- un transistor en série avec chaque capteur (12-22-13-23-14-24) adapté pour sélectionner le capteur (12-22-13-23-14-24) correspondant ;
- une résistance NTC adaptée pour connecter l'alimentation à un diviseur résistif (66) ;
- ledit diviseur résistif (66) étant adapté pour acquérir le courant à partir de ladite résistance NTC et générer une tension ;
- un LNA (67) adapté pour mettre à l'échelle et traduire les tensions de sortie possibles dudit diviseur résistif (66) afin d'exploiter pleinement la dynamique d'entrée dudit convertisseur analogique numérique (64).

7. Système pour comparer les paramètres physiologiques d'un patient, selon l'une des revendications précédentes, **caractérisé en ce qu'**au moins ledit dispositif de détection primaire (10) comprend des moyens d'interface USB (62) adaptés pour transmettre les données numérisées à d'autres dispositifs périphériques.

8. Système pour comparer les paramètres physiologiques d'un patient, selon l'une des revendications précédentes, **caractérisé en ce que** lesdits supports flexibles desdits dispositifs de détection (10-20-20'-...) sont en polyimide de manière à être adaptés à toute courbe du corps du patient.
